# EUROPEAN PATENT APPLICATION

(11) **EP 2 377 946 A1**
(43) Date of publication of application: **19.10.2011**
(21) Application number: 09836114.0
(22) Date of filing: 22.12.2009
(51) Int. Cl.: C12P 21/02, C12N 15/62, C07K 14/00

(54) **INCLUSION BODIES, BACTERIAL CELLS AND COMPOSITIONS CONTAINING THEM AND USES THEREOF**

(30) Priority: 30.12.2008 ES 200900045
(71) Applicant: Centro de Investigación Biomédica en Red en Bioingeniería, Biomateriales y Nanomedicina (CIBER BBN), 50018 Zaragoza (ES); Universitat Autònoma De Barcelona, 08193 Barcelona (ES); Centro de Investigación Biomédica en Red en Bioingeniería, Biomateriales y Nanomedicina (CIBER BBN), 50018 Zaragoza (ES)
(72) Inventor: VECIANA MIRO, Jaume, E-28006 Madrid (ES); RATERA BASTARDAS, Inmaculada, E-28006 Madrid (ES); DÍEZ GIL, César, E-28006 Madrid (ES); VILLAVERDE CORRALES, Antonio Pedro, E-08193 Bellaterra, Barcelona (ES); VÁZQUEZ GÓMEZ, Esther, E-08193 Bellaterra, Barcelona (ES); GARCÍA FRUITÓS, Elena, E-50018 Zaragoza (ES)
(74) Representative: Ponti Sales, Adelaida
(86) International application number: PCT/ES2009/070616
(87) International publication number: WO 2010/076361

(57) **Abstract**

The present invention refers to an isolated inclusion body comprising a polypeptide **characterised in that** the inclusion body is particulate in form. The present invention also refers to a bacterial cell comprising said inclusion body. The present invention additionally refers to a composition comprising said inclusion body and a eukaryotic cell. The present invention also refers to a composition comprising said inclusion body and an animal or plant tissue. The present invention also refers to the uses of said inclusion bodies as medicines and stimulators of cell proliferation and tissue regeneration.

## Description

### Field of the invention

The present invention refers to inclusion bodies, to bacterial cells and compositions containing them and their use as medicines and stimulators of cell proliferation and tissue regeneration.

### Background of the invention

Bacterial inclusion bodies (IBs) are highly pure protein deposits produced in recombinant bacterial. Being insoluble in water, they are observed as porous amorphous particles, highly hydrated, in the size range of several hundred nanometres. The polypeptide chains making up the IBs are folded in an amyloid-type molecular structure compatible with their native structure, thereby maintaining the biological activities of the included polypeptides (for example fluorescence or enzymatic activity). Therefore, after their appropriate manipulation, there is a wide spectrum of potential uses of IBs as functional and biocompatible materials. The biophysical characteristics of these proteinaceous particles such as activity and size have never been manipulated although this is theoretically feasible by the adjustment of genetic and production conditions. In this study, the nanoscale properties of IBs as new particulate materials are characterised and the degree to which the particles produced can be designed by simple strategies are explored. In addition, as a highlighted proof of concept, surfaces modified with inclusion bodies that significantly stimulate the proliferation of mammalian cells have been obtained, demonstrating the potential of IBs in tissue manipulation and regenerative medicine among other promising biomedical applications.

Many recombinant polypeptides produced in genetically modified bacteria aggregate as IBs. These protein deposits appear as highly hydrated particles found in bacterial cytoplasm² or, in some cases, in the periplasm³. The IBs are chemically pure as the recombinant protein is the main component, up to around 95% of the total protein^{2,4,5}. Other cellular molecules such as RNA, DNA and lipids are trapped during the formation of IBs and are present in minor quantities⁶. The formation of IBs is a rapid and effective process as they can be observed a few minutes after the induction of genetic expression. A few hours later, they can easily account for around 50% of the total cellular biomass⁵. Although it was believed in the past that IBs were formed by unfolded or substantially poorly folded polypeptide chains, and were therefore biologically inert, recent observations describe these particles as being made up of correctly folded proteins and, therefore, biofunctional⁸. The molecular structure of IBs is based on a particular amyloid-type organisation^{9,10} that allows crossed-linked beta sheet interactions to coexist with domains of correctly folded proteins¹¹. Therefore the IBs formed by enzymes can be useful catalysts in various types of bioprocesses such as those recently observed for proteins such as β-galactosidase, D-aminoacid oxidase, maltodextrine phosphorylase, sialic acid aldolase and polyphosphate kinase¹¹⁻¹⁵. Their formation in vivo, which implies deposition of proteins depending on the sequence around nucleation centres, is regulated by various cellular genes (mainly coding for proteases and chaperones that act as a functional network, which enables the manipulation of their properties at a nanoscale level)^{16,17}. The present invention determines the most important characteristics of IBs as nanoparticles and demonstrates that they can be designed by genetic manipulation and by manipulation of the appropriate process in the producing bacteria. Being totally biocompatible and mechanically stable materials, IBs have also been used as nanoparticles to modify the roughness of surfaces for stimulation of proliferation of mammalian cells. Given that essentially any protein species can be produced as bacterial IBs and their nanoscale properties can be easily adjusted, the functional possibilities of these novel materials offer an unusual spectrum of additional biomedical applications apart from those shown here in the context of tissue manipulation.

### Summary of the invention

The present invention refers to inclusion bodies, to bacterial cells and compositions containing them and their use as stimulators of cell proliferation and tissue regeneration.

A first object of the present invention refers to an isolated inclusion body comprising a polypeptide characterised in that the inclusion body is in particulate form.

A second object of the present invention refers to a bacterial cell comprising the inclusion body according to the first object of the invention and its different structural aspects.

A third object of the present invention refers to a composition comprising the inclusion body according to the first object of the invention and its different structural aspects and a eukaryotic cell.

A fourth object of the present invention refers to a composition comprising the inclusion body according to the first object of the invention and its different structural aspects and an animal or plant tissue.

A fifth object of the present invention refers to the uses of the inclusion body according to the first object of the invention and its different structural aspects and its disposal as a medicine and stimulator of cell proliferation and tissue regeneration.

### Brief description of the figures

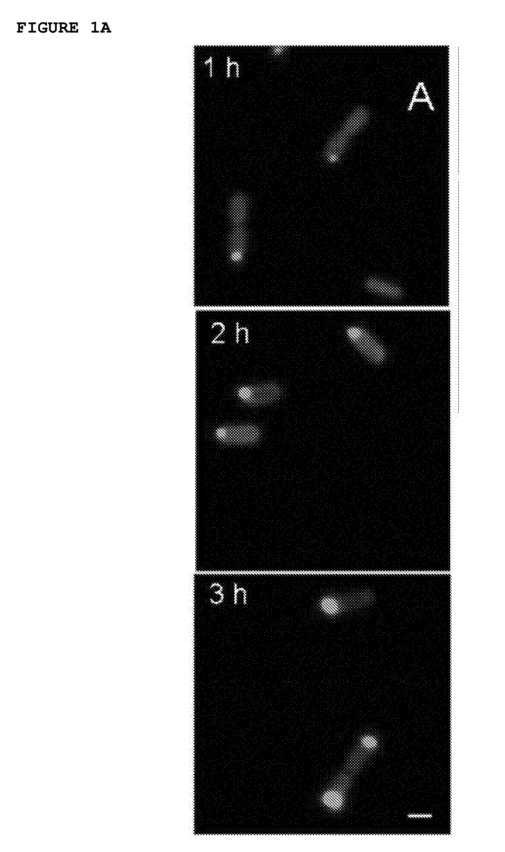

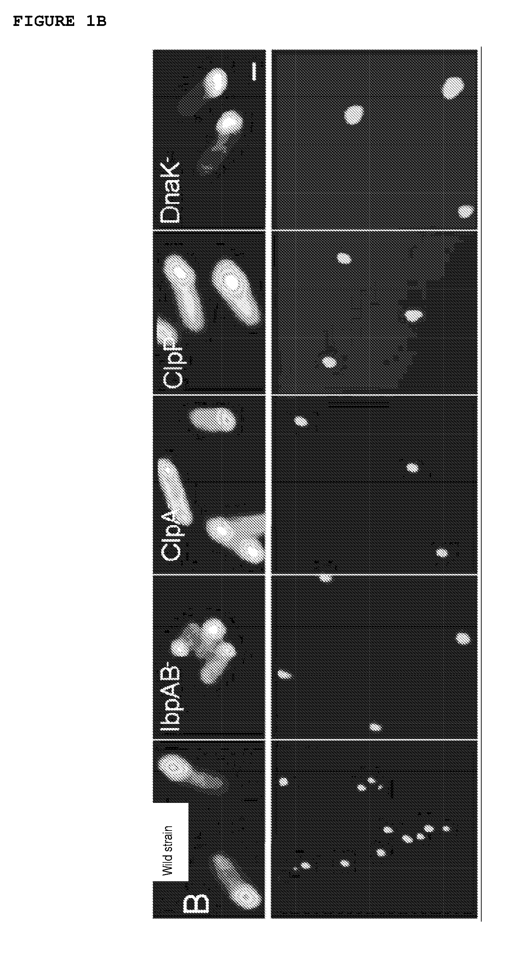

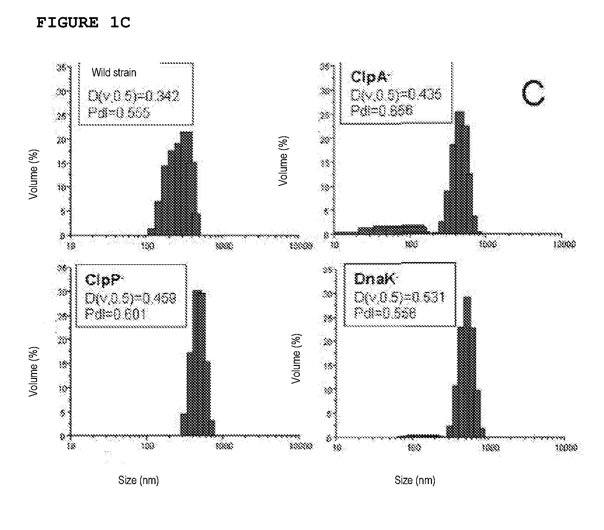

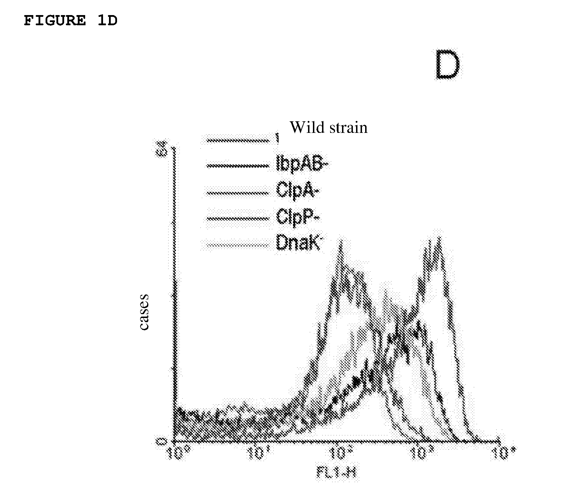

**Figure 1****. Morphological and functional characterisation of IBs. A.** Confocal microscopy images of wild bacterial cells producing IBs formed by a GFP fusion protein. From top to bottom, samples of cells taken at 1 hour, 2 hours and 3 hours after the induction of the production of the IB. **B.** Confocal microscopy images using a Metamorph's pallet of wild bacterial cells, IbpAB⁻, ClpA⁻, ClpP⁻ and DnaK⁻ producing IBs formed by a GFP fusion protein 2 hours after formation (upper section). Confocal microscopy images of IBs after 3 hours purified from these strains (lower section). The bars in A and B indicate 1 µm. **C.** Distribution curves of particle size measured by dynamic light scattering of IBs after 3 hours after formation produced by different bacterial strains. The IBs of cells deficient in IbpAB were excluded from this study due to their particular tendency to aggregate as supraparticle complexes (results not shown). The curves are represented in terms of the percentage in volume of the particles. The distributions of particle volumetric size are described as D[v,0.5], which is the particle diameter (nm) below which there is 50% of the total system volume. D[v,0.5] is the mean particle diameter (nm) in the volume. The polydispersity index (PdI) is defined as [D(v,0.1)/D(v,0.9)]·100. **D.** Fluorescence emitted by IBs analysed by flow cytometry for particles purified from *E. coli* strains IbpAB⁻, ClpA⁻, ClpP⁻ and DnaK⁻.

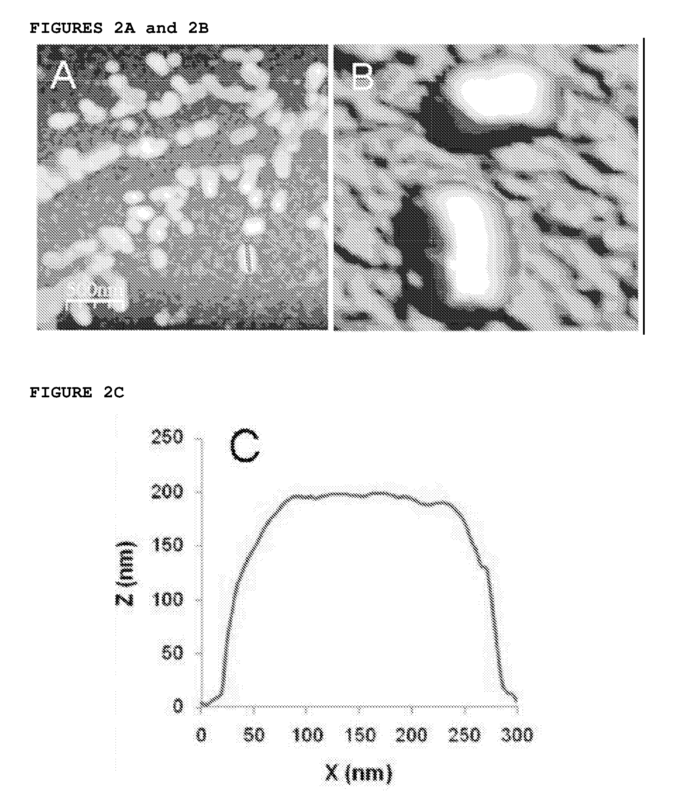

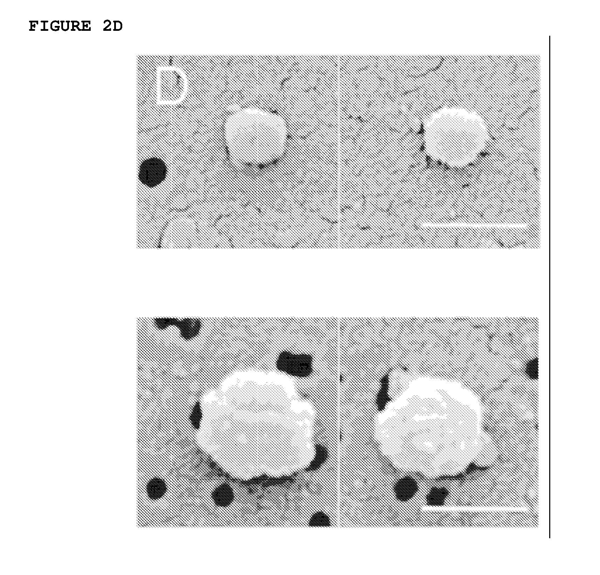

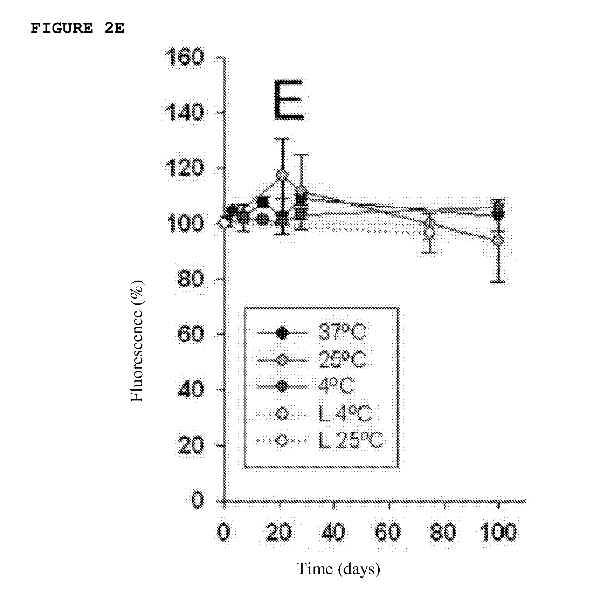

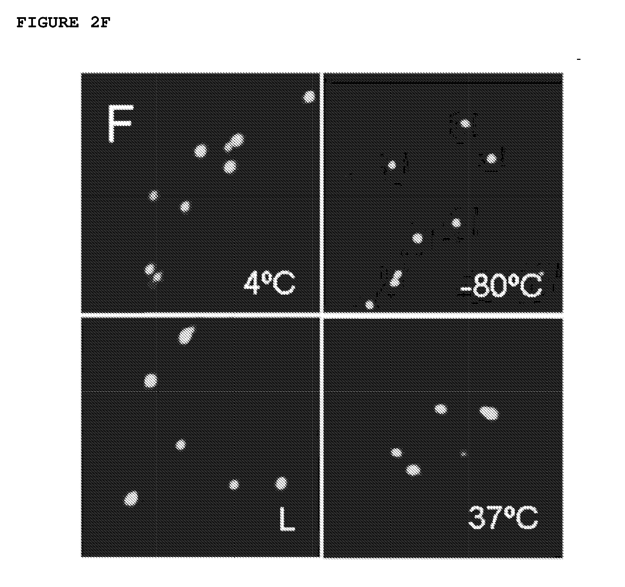

**Figure 2****. Fine structure and stability of IBs with GFP.** In the upper images, IBs made up of a GFP fusion protein 3 hours after formation, characterised by Atomic Force Microscopy (AFM) are shown: **A.** Topographic image of 2.5 x 2.5 µm of IBs randomly deposited on the surface. **B.** 3D image of 600 x 600 nm showing two IBs of the image of panel A. **C.** Topographical cross section of a particle of isolated IB (indicated as a blue line in A), which indicates the existence of a certain RMS intrinsic roughness on the surface of the IB of 1.89 nm as a consequence of the internal structure, which results in a fine surface texture. In **D,** SEM images of IBs 3 hours after formation produced in wild cells (upper section) and in DnaK⁻ cells (lower section). The white bars indicate 500 nm. In **E** the stabilities of IBs in aqueous buffer at 37°C, 25°C and 4°C, or lyophilised (L) and later stored at 25°C or 4°C are shown. **F.** Confocal microscopy images of purified IBs maintained in buffer for one month at 25°C, 4°C and -80°C, and after lyophilisation/reconstitution (L).

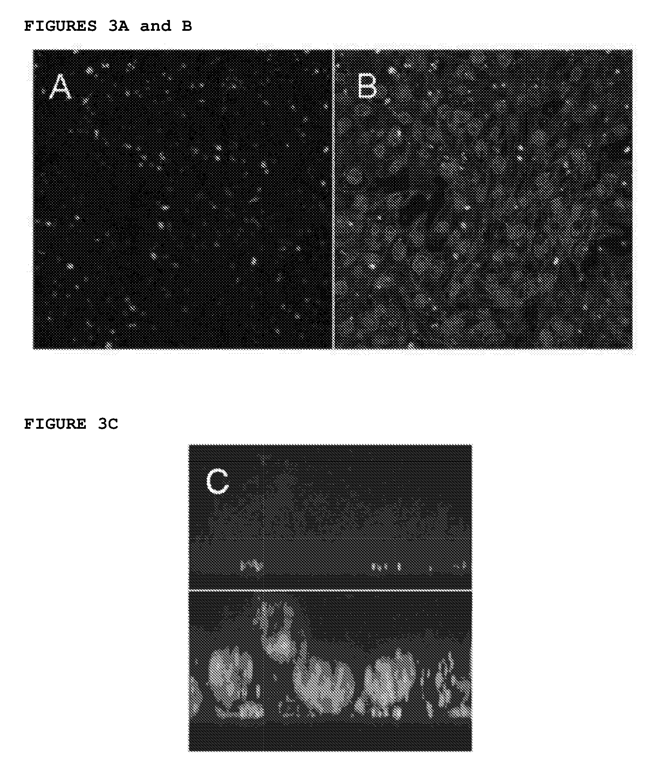

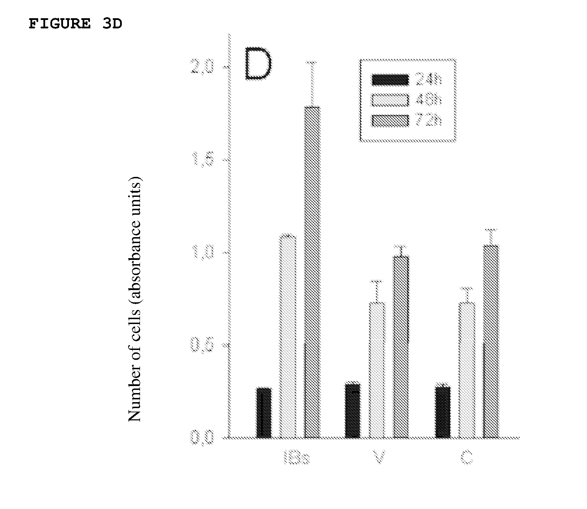

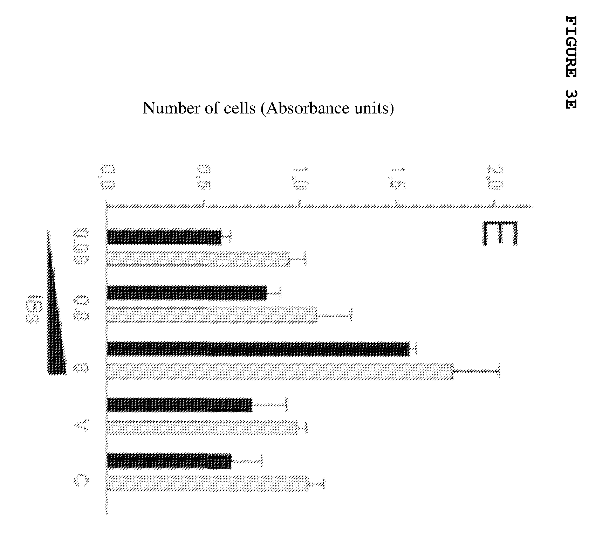

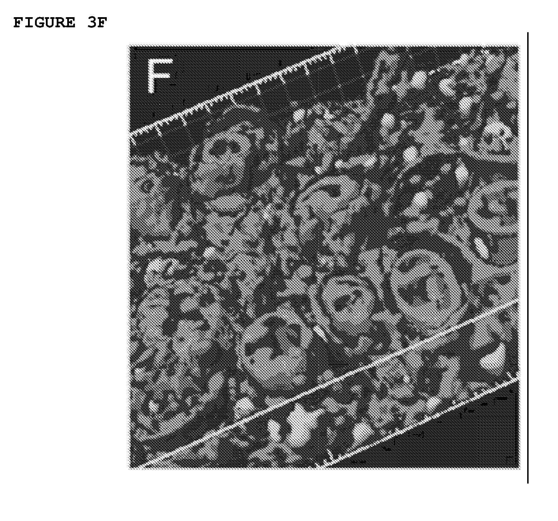

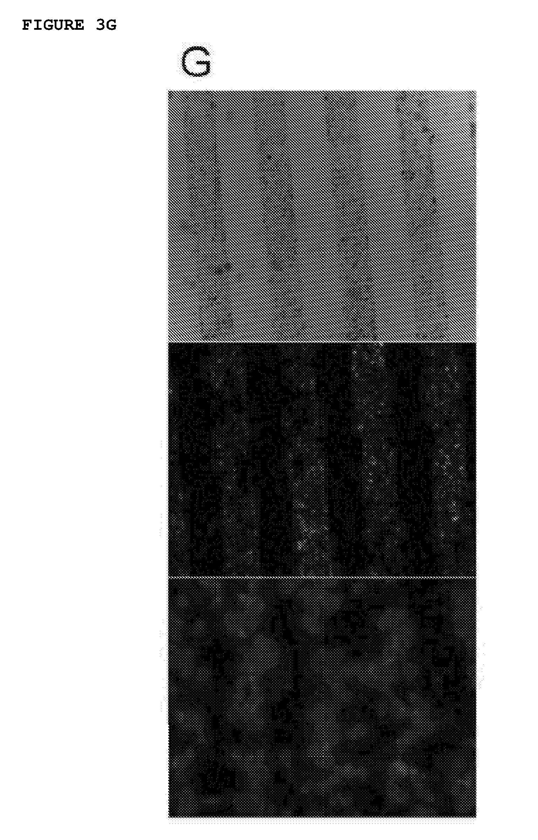

**Figure 3****. Mammalian cell proliferation stimulated by IBs.** A. Confocal image of a 35 mm polystyrene plate covered with 240 µg of IBs with GFP produced in natural cells. B. For the same field, the covering of BHK cells of the 0.6 µm section 75 hours after cell deposition and a xzy projection of IBs formed by a GFP fusion protein (upper section) and cell covering (lower section). **C.** Cellular nuclei stained with Hoechst 33342 and cellular membranes with CellMask immediately before being analysed by confocal laser scanning microscopy of three sequential channels. In **D,** growth of BHK cells at different incubation times on plates covered with IBs (IB), on plates covered with vitronectin (V) and on control plates (C). **E.** Growth of BHK cells on plates covered with different concentrations of IBs compared to plates covered with vitronectin (V) and control plates (C). The experiments were carried out in parallel on culture treated polystyrene plates (black bars) and untreated plates (grey bars). **F.** xyz confocal stack of 22 sections processed with Imaris 3D software by the application of the Isosurface module. **G.** Images of the surfaces of amino-terminated silicon stamped with 50 µm coating of IBs taken via conventional microscopy (upper section) and confocal microscopy (middle section) and the distribution of BHK cells after 48 hours of growth on the surfaces (lower section).

### Description of the invention

The present invention refers to inclusion bodies, bacterial cells and compositions containing them and their use as stimulators of cell proliferation and tissue regeneration.

The term "inclusion body", also denominated in the present invention as "IB" is understood as previously indicated in the background section, or in a simpler way, as an amorphous intracellular deposit comprising aggregated proteins that are found in the cytoplasm of a cell.

A first object of the present invention refers to an isolated inclusion body comprising a polypeptide characterised in that the inclusion body is in particulate form.

In a preferred embodiment, the particulate form has a particle size of between 24 and 1500 nm.

In a more preferred embodiment, the particle is in a hydrated amorphous form.

With respect to the polypeptide comprising the inclusion body according to the first object of the present invention, this can be a chimeric polypeptide comprising a viral protein translationally fused to a reporter protein.

In a preferred embodiment, said viral protein is a capsid protein.

In addition, in another preferred embodiment, the reporter protein is a fluorescent protein. In particular, said fluorescent protein is GFP (green fluorescent protein).

In a particular embodiment, the inclusion body according to the first object of the invention and its different structural aspects is deposited on a plate treated with a tissue culture medium.

In another particular embodiment, the inclusion body according to the first object of the invention and its different structural aspects is deposited on a silicon substrate.

In still another particular embodiment, the inclusion body according to the first object of the invention and its different structural aspects is incorporated within a three-dimensional synthetic or natural framework.

A second object of the present invention refers to a bacterial cell comprising the inclusion body according to the first object of the invention and its different structural aspects.

In a preferred embodiment, said bacterial cell is *Escherichia Coli* (*E. coli*).

In a more preferred embodiment, said bacterial cell of *E. coli* is a wild strain (WT) or is a mutant strain.

A third object of the present invention refers to a composition comprising the inclusion body according to the first object of the invention and its different structural aspects and a eukaryotic cell.

In a preferred embodiment, said eukaryotic cell is a mammalian cell.

A fourth object of the present invention refers to a composition comprising the inclusion body according to the first object of the invention and its different structural aspects and an animal or plant tissue.

A fifth object of the present invention refers to the uses of the inclusion body according to the first object of the invention and its different structural aspects and disposition.

A first use of said inclusion body is as a stimulator of the proliferation of eukaryotic cells.

A second use of said inclusion body is as a tissue regenerator.

A third use of said inclusion body is as a medicine. In particular, said use as a medicine would take into account the advantages observed (see experimental section) in cell proliferation and tissue regeneration.

The following examples are offered only for illustration purposes and do not limit the scope of the present invention in any way.

### Materials and methods

### Bacterial cells, plasmids and the production of inclusion bodies

The IBs are produced in different strains of *Escherichia coli*, specifically MC4100 (wild strain with respect to folding and degradation of proteins, araD139 Δ*(argF-lac) U169 rpsL150 relA1 flbB5301 deoC1 ptsF25 rbsR*)¹⁸ and its derivatives JGT4 (deficient in co-protease ClpA, *clpA::kan*), JGT17 (deficient in the small thermal shock proteins IbpAB, Δ*ibp::kan*), JGT19 (deficient in the co-protease ClpP, *clpP::cat*) and JGT20 (deficient in the main chaperone DnaK, *dnak756 thr::Tn10*)¹⁹. These producing strains were transformed with the expression vector pTVPIGFP (Ap^{R}) which codes for the green fluorescent protein (GFP) fused at the amino terminal to VP1, the pentameric form of the capsid protein of aphthous fever virus²⁰ This viral protein, being highly hydrophobic, directs the deposition of fusion proteins as IBs⁹. The recombinant gene is expressed under the control of an inducible *trc* promotor by isopropyl β-D-1-thiogalactopyranoside (IPTG).

The bacterial cells were cultured in LB rich medium¹⁸ and the fusion gene was expressed under standard conditions as previously described¹⁷. The IBs were clearly detected 1 hour after addition of IPTG (Figure 1A).

### Purification of the inclusion bodies

Samples of 200 ml bacterial culture at 4°C were centrifuged at 5000 g for 5 minutes and resuspended in 50 ml of lysis buffer (50 mM Tris-HCl pH 8.1, 100 mM NaCl and 1mM EDTA). The samples maintained in ice were sonicated (for 25 to 40 minutes) at 40% amplitude under 0.5 s cycles. After sonication, 28 µl of 100 mM phenylmethylsulfonyl fluoride (PMSF) and 23 µl of 50 mg/ml lysozyme were added to the samples, which were incubated at 37°C under stirring for 45 minutes. Next, 40 µl of Nonidet P40 (NP-40) were added and the mixture stirred for 1 hour at 4°C. The DNA was removed with 120 µl of 1 mg/ml DNAase and 120 µl of 1M Mg₂SO for 45 minutes at 37°C under stirring. Finally, the samples were centrifuged at 4°C at 15,000 g for 15 minutes and the residue, containing pure IBs, was washed with lysis buffer containing 0.5% Triton X-100 and was kept at -20°C until analysis.

### Microscopic analysis of bacteria and IBs

Samples were analysed using a Leica TSC SP2 AOBS (Leica Microsystems Heidelberg GMBH, Manheim, Germany) confocal fluorescence microscope after excitation at a wavelength of 488 nm and the images were recorded at emission wavelengths of between 500 and 600 nm (63X, 1.4 NA oil) using a Plan-Apochromat (zoom 8; 1024 by 1024 pixels) objective. For the analysis of the bacterial cells producing fluorescent IBs, samples taken at 1, 2 and 3 hours after induction by IPTG were fixed with 0.2% formaldehyde in phosphate buffered saline (PBS) and kept at 4°C until use. The isolated IBs were resuspended in 20 ml PBS. For scanning electronic microscopy (SEM), samples were analysed by conventional procedures using a FEG (field emission gun)-ESEM (environmental scanning electron microscope).

### Flow cytometry

The purified IBs were resuspended in PBS and sonicated for 4 minutes under 0.5 s cycles and were analysed by flow cytometry in a FACS Calibur (Becton Dickinson) system using an air-cooled 15 mW argon ion laser at an excitation wavelength of 488 nm. The IB fluorescence emission was measured in the FL-1 channel (530/30 nm band pass filter) using a logarithmic mode.

### Characterisation by atomic force microscopy

Atomic force microscopy (AFM) analysis was carried out in air with a commercial atomic force microscope (PicoScan/PicoSPM from Molecular Imaging Agilent Technologies, Inc., Santa Clara, CA, USA) operating in acoustic mode. The IBs resuspended in 0.1 M phosphate buffer at pH 7.4 were deposited on a mica surface and were dried in air before measurement. For measurements in acoustic mode, a PPP-NHC (Nanosensors, Inc.) monolithic silicon probe was used with a nominal spring constant of 42 N/m and a resonance frequency of 330 kHz.

### Dynamic light scattering measurements

Size distributions in a volume of IB and the Zeta potential were measured using a dynamic light scattering (DLS) analyser at a wavelength of 633 nm, combined with non-invasive back-scatter (NIBS) technology (Zetasizer Nano ZS, Malvern Instruments Limited, Malvern, United Kingdom). Dispersions of IBs of 3 hours life in 0.1 M phosphate buffer at pH 7.4 (500 ng/µl) were prepared by brief sonication (1 minute at room temperature). Aliquots of 3 ml of the resulting dispersion at 20°C were measured without filtering before measurement. The intensity data were normalised using 0.1 M phosphate buffer at pH 7.4 as a reference standard. The average value of three different measurements was taken as the average IB hydrodynamic diameter.

### Preparation of amino-terminated monolayers

Silicon substrates (100) 1x1 cm polished on one side were used for the preparation of amino-terminated monolayers. Before the formation of the monolayer, the substrates were treated with a RCA-1 oxidising solution (NH₄OH/H₂O₂/H₂O in a ratio 1:1:5) for 30 minutes at 80°C and gently rinsed with ultrapure/MilliQ water with a conductivity higher than 18.2 MΩ. Subsequently, the substrates were introduced into pyranha solution (concentrated H₂SO₄ (Panreac) and aqueous 33% H₂O₂ (Aldrich) in a proportion of 3:1) for 15 minutes, rinsed abundantly with ultrapure water and dried in a stream of nitrogen. This treatment provides a new surface on the substrates terminated in hydroxyl groups for later reactions. The amino-terminated monolayers were formed by exposure of the substrates under controlled atmosphere to a solution of 5 mM N-[3-(trimethoxysilyl)propyl]ethylenediamine (TPEDA) (97% Aldrich) in anhydrous toluene for 3 hours. After forming the monolayer, the substrates were rinsed with toluene and ethanol to remove excess silane and were dried in a stream of nitrogen. The angle of contact of the amino-terminated substrate was measured with a drop of 3 µl of ultrapure water (MilliQ with 18.2 MΩ cm) in a OCA15+ (Data Physics Instruments GMBH, Germany) angle of contact measurement instrument equipped with a CCD camera and SCA20 software for angle determination. The XPS spectra were obtained in a PHI ESCA-500 (Perkin Elmer) instrument equipped with a monochromatic Al K-alpha X-ray source operating at 350 W. The spectra made reference to the main C1s peak observed at 284.8 eV.

### Microcontact printing (µCP) of IBs on the amino-terminated silicon substrate

The µCP of IBs on the amino-terminated silicon substrate was performed using PDMS stampings (Sylgard 184, Dow Corning, United States). The stampings were made by melting a 10:1 (v/v) mixture of PDMS and curing agent (Sylgard 184, Dow Corning) against a silicon base with a photolithographic pattern, curing for 1 hour at 60°C and were extracted at this curing temperature. The PDMS stampings were left in the oven at 60°C for a period of at least 18 hours to ensure complete curing. For IBs printed with µCP, the PDMS stampings were impregnated with a suspension of IBs in PBS buffer (pH 7.5) for 40 minutes, dried in a stream of nitrogen and placed on a surface of clean amino-terminated silicon substrate. After a contact time of 1 minute, the stamping was carefully removed.

Fluorescence of the printed samples was analysed using a Leica TSC SPE (Leica Microsystems Heidelberg GMBH, Manheim, Germany) confocal fluorescence microscope using excitation at a wavelength of 488 nm and measuring emission between 500 and 600 nm (x 10 air).

### Stability analysis

The IBs formed in DnaK⁻ cells after 5 hours were diluted in PBS with 10 g/l bovine serum albumin (BSA) and 60 g/l sucrose in the presence of 40 mg/l gentamicin, 100 U/ml penicillin and 10 µg/ml streptomycin and aliquots were incubated at different temperatures (37°C, 25°C or 4°C). Samples were frozen at different times at -80°C for fluorescence determination. Fluorescence was recorded at 510 nm in a Cary Eclipse (Variant, Inc., Palo Alto, CA) fluorescence spectrophotometer using an excitation wavelength of 450 nm. The results are referred to the percentage of activity or remaining fluorescence compared to control samples maintained at -80°C, which were fully stable. Another group of samples were lyophilized in a Telstar Cryodos-80 freeze-dryer and were stored at 4°C or 25°C until analysis.

### Cell proliferation test

Isolated inclusion bodies containing GFP as described above were sterilised after 3 hours of production by exposure to a germicidal UV lamp at a wavelength of 253 nm for 4 hours. They were then resuspended in PBS and different amounts of IB protein, specifically 0.08, 0.8 and 8 µg per well, were used to cover Falcon 3072 96-well polystyrene plates treated with tissue culture medium (Becton Dickinson) or untreated Costar 3370 plates and incubated overnight at 4°C. Vitronectin (Calbiochem) was used as reference at a concentration of 50 ng/cm², following the manufacturer's instructions. The wells were washed in PBS and blocked with 3% BSA in PBS for 1 hour at 37°C. 1.5-10² cells of a newborn hamster kidney (BHK) cell line were then added per well and incubated in Dulbecco's modified Eagle's medium (DMEM) supplemented with non-essential amino acids, 5% foetal calf serum, gentamicin and antimycotics at 37°C for different times. Control wells followed exactly the same treatment described above but were maintained without IBs.

After incubation, cell proliferation was determined using the EZ4U kit (Biomedica, GMBH) following the manufacturer's instructions and were analysed in the VICTOR³ V Multilabel Counter model 1420 (Perkin Elmer). The reading absorbances were 450 nm and 620 nm as reference and the values obtained were standardised with respect to the wells only containing medium. A pre-test was carried out to select the incubation time before saturation with the kit reagents; the optimum times were 3 hours for 24-hour cultures, 2 hours for 48-hour cultures and 30 min for 72-hour cultures. All the tests were performed in triplicate. The data were expressed as mean ± SEM of the values of the three experiments carried out for each condition and were statistically evaluated using an ANOVA test, followed by post-hoc Bonferroni analysis. The significance level was p < 0.05. For cell growth on silicon, the surfaces with IB grafts were cut into suitable sizes, irradiated with UV and deposited in Falcon 24-well polystyrene plates with tissue culture medium where the cells were inoculated and cultured using conventional procedures.

### Analysis of cell cultures by confocal microscopy

Cell cultures were examined using a Leica TCS SP5 AOBS confocal spectral microscope (Leica Microsystems, Mannheim, Germany) using a Plan-Apochromat 63X 1.4 NA lens. All the images were obtained from live cells grown on glass-bottom dishes (MatTek Corporation, Ashland, MA, USA). The cells were seeded at a density of 4 x 10⁴ /well of inclusion bodies with GFP, 72 hours before the collection and culture in DMEM + Glutamax 1 (Gibco) supplemented with 10% foetal serum albumin. For marking the nuclear and plasma membrane, the cells were incubated with 5 µg/ml of Hoechst 33342 and 5 µg/ml of CellMask (both from Molecular Probes, Inc., Eugene, OR, United States) respectively for 5 minutes at room temperature and washed twice before confocal detection. The nuclei were excited with a laser diode beam at 405 nm and observed at 414-461 nm (blue channel); the plasma membrane was detected by excitation with helium and neon laser light at 633 nm and fluorescence observed at 656-789 nm (infra red channel); finally a line of 488 nm from an argon laser was used to obtain images of IBs (green channel, emission at 500-537 nm). The Zeta series of 22 optical sections were collected at an interval of 0.6 µm. The Zeta-layers were obtained with the LAS AF (Leica Microsystems) software and three-dimensional models were generated using the Imaris software (Bitplane, Zurich, Switzerland).

### Results and discussion

IBs formed by green fluorescent protein (GFP) are convenient models for kinetic and functional analysis of their biological production because they are highly fluorescent²⁰ After the addition of IPTG, a lactose analogue, to the bacterial culture, the IBs are clearly visible by confocal microscopy 1 hour after induction of the expression of the GFP gene (Figure 1A) and grow volumetrically until around 3 hours during the synthesis of recombinant GFP, enabling the collection of IBs in a broad size spectrum. Under standard conditions for bacterial growth in the laboratory without any attempt to optimise, production was greater than 5 mg/l after 3 hours (result not shown), a very promising yield for a practical scale. In addition, different strains of *E. coli*, deficient in chaperones or proteases, produce IBs of different sizes (Figure 1B) with the same yield due to the different dynamics of the deposition of proteins *in vivo* therein^{16,17}. Although mature IBs purified from natural cells showed an average diameter of 340 nm (compatible with independent estimates²¹), this value can be progressively increased by the use of mutant cells to more than 500 nm (in DnaK⁻ deficient cells) (Figure 1C), with a relatively low polydispersity index in all analysed samples. Particle fluorescence emission was also determined in purified IBs by flow cytometry and a defined interval from nanoparticles with low fluorescence (natural strain) to nanoparticles with high fluorescence (ClpP⁻ mutant strain) was observed (Figure 1D). Next, the appropriate combination between the collection time during the production process (which determines the stage of growth of the IB) and the producing strain (which determines both biological activity and the upper size limit) would define the particle dimensions and specific fluorescence that could be optimised for different applications. For example, the IBs obtained in ClpA⁻ and ClpP⁻ cells, with a very similar particle size (0.435 and 0.459 nm respectively) showed different fluorescence emission levels (average 71 and 184 FL1 units per particle respectively). However, the mapping of the fluorescence of IBs with GFP were comparable in all strains, showing a common central homogenous fluorescence pattern (Figure 1B).

In order to further characterise the nanoscale morphology of the IBs, they were investigated by AFM and SEM. As shown in Figure 2A,B,C the AFM of natural IBs with GFP indicates the presence of isolated particles with spherical or cylindrical shape with average sizes of 300 nm length, 170 nm diameter and 200 nm height. Cross section measurements performed confirmed the statistical data obtained from DLS measurements (Figure 1C). The SEM observations were in line with the AFM images, revealing that IBs have a rough surface and accentuating the size difference between IBs obtained in natural and DnaK⁻ cells (Figure 2D). Measurements of the Zeta potential performed in a fresh IB suspension showed a value of -9.8 mV which is indicative of a slightly negatively charged surface and is in agreement with the trend of the proteins to form aggregates. On the other hand, the stability of IBs under conditions commonly used for storing biological samples was also investigated. IBs were found to be completely stable over long periods with respect to both fluorescence emission and structure at -80°C, 4°C, 25°C and also at 37°C (Figures 2E,F), which permits not only storage but also convenient use and manipulation of IBs under physiological test conditions. Interestingly, IBs were also mechanically and functionally stable during lyophilisation (and under various additional storage conditions, Figure 2F) and sonication (not shown), expanding their potential uses under diverse experimental conditions.

Given that bacterial IBs are easily manipulated and are fully biocompatible materials, their potential applicability for biomedical purposes was investigated by a simple exercise. In the generation of tissues for regenerative medicine, cell binding and proliferation can be stimulated by topographical modification of the properties of a material surface by printing, lithography and similar procedures, depending on the nature of the material. Recently, other strategies based on the functionalisation of surfaces with materials^{22,23,24,25} or in decoration of surfaces with nanoparticles also allows fine adjustment of surface texture and roughness independently of the nature of the material used to stimulate cell binding^{26,27}. In this context, silica nanoparticles and ceramics of between 24 and 1500 nm in diameter affect cell growth functions and can positively modulate cell proliferation on decorated surfaces^{24,26}. Given that we wanted to find out whether bacterial IBs appearing in this size interval could also be useful for surface nanomanipulation, we tested the effect of IBs with GFP deposited on polystyrene plates treated with tissue culture medium (Figure 3A) on the growth of BHK21 cells (Figures 3B, C). At a density of 0.05 particles/µm², IBs had a root mean square (RMS) roughness of 55.9 nm. On said modified surface, BHK21 cells grew intimately bound to deposited IBs with GFP, as was observed by the appearance of stain in the cellular membrane (red) and IB fluorescence (green) resulting in yellow signals (Figure 3B). Suprisingly, on a polystyrene surface, which is broadly optimised for cell adhesion and growth, a conventional cellular binding agent such as vitronectin did not show detectable effects on cell proliferation (Figures 3D, E). However, under these favourable conditions, the IBs still significantly stimulated cell proliferation (Figure 3D) depending on the dose (Figure 3E) of up to more than 2-fold. This effect was much more pronounced and biologically significant than the slight or zero effects observed with other nanoparticles, which at defined sizes and for some cell lines seemed to show inhibitory effects instead of stimulating effects^{26,28,29}. Interestingly, cytopathic or toxicity symptoms were not observed on cultured cells after growing on surfaces treated with IBs. Analysis of confocal 3D images (Figure 3F) showed IBs bound to polystyrene to be totally integrated in the cellular membranes, indicating an intimate interaction between the cellular surfaces and the IB nanoparticles decorating the cellular growth surface.

In order to further demonstrate the validity of IBs as cell proliferation stimulators, microstructuration of IBs was performed on an amino-terminated silicon substrate^{30,31} using the micro-contact printing technique (µCP), which involves the impregnation of an elastomeric stamp with a suspension of IBs. Figure 3G shows the silicon surface stamped with IBs at a density of 0.04 IBs/µm² and a RMS roughness of 32.4 nm as well as the consequent stimulation of cell proliferation in regions linearly decorated with IBs. This indicates the preference for cell growth induced by IBs and the capacity of these nanoparticles to stimulate cell proliferation on surfaces initially not appropriate for cell growth.

In summary, IBs produced in bacteria can be designed with precision during biological production with respect to important characteristics at a nanoscale level and are fascinating nanoparticulate materials produced by economical processes in biological systems. Being biofunctional by nature and given that the protein of which they are formed can be selected and that its biological activity can be modulated by genetic modification of the producing cell, manipulation of IBs could have broad and profound implications in different nanomedical fields. In particular, and as a first proof of concept of biomedical applicability, IBs functionalize effectively surfaces for significantly encouraging the proliferation of bound mammalian cells.

### References:

1. Villaverde, A. & Carrio, M. M. Protein aggregation in recombinant bacteria: biological role of inclusion bodies. Biotechnol Lett 25, 1385-1395 (2003).
2. Carrio, M. M., Cubarsi, R. & Villaverde, A. Fine architecture of bacterial inclusion bodies. FEBS Lett 471, 7-11 (2000).
3. Arie, J. P., Miot, M., Sassoon, N. & Betton, J. M. Formation of active inclusion bodies in the periplasm of Escherichia coli. Mol. Microbiol. 62, 427-437 (2006).
4. Bowden, G. A., Paredes, A. M. & Georgiou, G. Structure and morphology of protein inclusion bodies in Escherichia coli. Biotechnology (N. Y. ) 9, 725-730 (1991).
5. Carrio, M. M., Corchero, J. L. & Villaverde, A. Dynamics of in vivo protein aggregation: building inclusion bodies in recombinant bacteria. FEMS Microbiol Lett 169, 9-15 (1998).
6. Neubauer, P., Fahnert, B., Lilie, h. & Villaverde, A., pp. 237-2922006).
7. Baneyx, F. & Mujacic, M. Recombinant protein folding and misfolding in Escherichia coli. Nat. Biotechnol. 22, 1399-1408 (2004).
8. Ventura, S. & Villaverde, A. Protein quality in bacterial inclusion bodies. Trends Biotechnol. 24, 179-185 (2006).
9. Carrio, M., Gonzalez-Montalban, N., Vera, A., Villaverde, A. & Ventura, S. Amyloid-like properties of bacterial inclusion bodies. J. Mol. Biol. 347, 1025-1037 (2005).
10. Wang, L., Maji, S. K., Sawaya, M. R., Eisenberg, D. & Riek, R. Bacterial inclusion bodies contain amyloid-like structure. PLoS. Biol. 6, e195 (2008).
11. Garcia-Fruitos, E., Aris, A. & Villaverde, A. Localization of functional polypeptides in bacterial inclusion bodies. Appl. Environ. Microbiol. 73, 289-294 (2007).
12. Nahalka, J., Dib, I. & Nidetzky, B. Encapsulation of Trigonopsis variabilis D-amino acid oxidase and fast comparison of the operational stabilities of free and immobilized preparations of the enzyme. Biotechnol. Bioeng. 99, 251-260 (2008).
13. Nahalka, J., Gemeiner, P., Bucko, M. & Wang, P. G. Bioenergy beads: a tool for regeneration of ATP/NTP in biocatalytic synthesis. Artif. Cells Blood Substit. Immobil. Biotechnol. 34, 515-521 (2006).
14. Nahalka, J., Vikartovska, A. & Hrabarova, E. A crosslinked inclusion body process for sialic acid synthesis. J. Biotechnol. 134, 146-153 (2008).
15. Nahalka, J. Physiological aggregation of maltodextrin phosphorylase from Pyrococcus furiosus and its application in a process of batch starch degradation to alpha-D: -glucose-1-phosphate. J. Ind. Microbiol. Biotechnol. 35, 219-223 (2008).
16. Carrio, M. M. & Villaverde, A. Role of molecular chaperones in inclusion body formation. FEBS Lett 537, 215-221 (2003).
17. Garcia-Fruitos, E. et al. Divergent Genetic Control of Protein Solubility and Conformational Quality in Escherichia coli. J. Mol. Biol. 374, 195-205 (2007).
18. Sambrook, J., Fritsch, E. & Maniatis, T. Molecular Cloning, A Laboratory Manual, Cold Spring Harbor Laboratory Press, Cold Spring Harbor, NY. 1989. Ref Type: Generic
19. Thomas, J. G. & Baneyx, F. Roles of the Escherichia coli small heat shock proteins IbpA and IbpB in thermal stress management: comparison with ClpA, ClpB, and HtpG In vivo. J Bacteriol 180, 5165-5172 (1998).
20. Garcia-Fruitos, E. et al. Aggregation as bacterial inclusion bodies does not imply inactivation of enzymes and fluorescent proteins. Microb. Cell Fact. 4, 27 (2005).
21. Margreiter, G., Messner, P., Caldwell, K. D. & Bayer, K. Size characterization of inclusion bodies by sedimentation field-flow fractionation. J. Biotechnol. 138, 67-73 (2008).
22. Suci, P. A., Klem, M. T., Arce, F. T., Douglas, T. & Young, M. Assembly of multilayer films incorporating a viral protein cage architecture. Langmuir 22, 8891-8896 (2006).
23. Ludden, M. J., Mulder, A., Tampe, R., Reinhoudt, D. N. & Huskens, J. Molecular printboards as a general platform for protein immobilization: a supramolecular solution to nonspecific adsorption. Angew. Chem. Int. Ed Engl. 46, 4104-4107 (2007).
24. Masaki Uchida, Michael T. Klem, Mark Allen, Peter Suci, Michelle Flenniken, Eric Gillitzer, Zachary Varpness, Lars O. Liepold, Mark Young, and Trevor Douglas, Biological Containers: Protein Cages as Multifunctional Nanoplatforms, Adv. Mater., 19, 1025-1042 (2007)
25. Dawn M. Yanker and Joshua A. Mauer, Direct printing of trichlorosilanes on glass for selective protein adsorption and cell growth, Mol. Biosyst., 4, 502-504 (2008).
26. Lipski, A. M., Pino, C. J., Haselton, F. R., Chen, I. W. & Shastri, V. P. The effect of silica nanoparticle-modified surfaces on cell morphology, cytoskeletal organization and function. Biomaterials (2008).
27. El-Ghannam, A. R. et al. Model surfaces engineered with nanoscale roughness and RGD tripeptides promote osteoblast activity. J. Biomed. Mater. Res. A 68, 615-627 (2004).
28. Dulgar-Tulloch, A. J., Bizios, R. & Siegel, R. W. Human mesenchymal stem cell adhesion and proliferation in response to ceramic chemistry and nanoscale topography. J. Biomed. Mater. Res. A (2008).
29. Samaroo, H. D., Lu, J. & Webster, T. J. Enhanced endothelial cell density on NiTi surfaces with submicron to nanometer roughness. Int. J. Nanomedicine. 3, 75-82 (2008).
30. Dorota I. Rozkiewicz, Bart Jan Ravoo, and David N. Reinhoudt Reversible Covalent Patterning of Self-Assembled Monolayers on Gold and Silicon Oxide Surfaces Langmuir, 21, 6337-6343 (2005).
31. Shu-Han Hsu, David N. Reinhoudt, Jurrian Huskens, and Aldrik H. Velders, Imidazolide monolayers for reactive microcontact printing, J. Mater. Chem., 18, 4959-4963 (2008).
32. Kane, R. S., Takayama, S., Ostuni, E., Ingber, D. E. & Whitesides, G. M. Patterning proteins and cells using soft lithography. Biomaterials 20, 2363-2376 (1999).
33. Renault, J. P. et al. Fabricating microarrays of functional proteins using affinity contact printing. Anew. Chem. Int. Ed Engl. 41, 2320-2323 (2002).

## Claims

1. Isolated inclusion body comprising a polypeptide **characterised in that** the inclusion body is in particulate form.

2. Isolated inclusion body according to claim 1, where the particulate form has a particle size of between 24 and 1500 nm.

3. Isolated inclusion body according to any of the claims 1-2, where the particle is in hydrated amorphous form.

4. Isolated inclusion body according to any of the claims 1-3, where the polypeptide is a chimeric polypeptide comprising a viral protein translationally fused to a reporter protein.

5. Isolated inclusion body according to claim 4, where the viral protein is a capsid protein.

6. Isolated inclusion body according to any of the claims 4-5, where the reporter protein is a fluorescent protein.

7. Composition comprising the inclusion body according to any of the claims 1-6 and a eukaryotic cell.

8. Composition according to claim 7, where the eukaryotic cell is a mammalian cell.

9. Composition comprising the inclusion body according to any of the claims 1-6 and an animal or plant tissue.

10. Isolated inclusion body according to any of the claims 1-6, where the inclusion body is deposited on a plate treated with tissue culture medium.

11. Isolated inclusion body according to any of the claims 1-6, where the inclusion body is deposited on a silicon substrate.

12. Isolated inclusion body according to any of the claims 1-6, where the inclusion body is incorporated within a synthetic or natural three-dimensional framework.

13. Use of the isolated inclusion body according to any of the claims 1-6 and 10-12, as a stimulator of eukaryotic cell proliferation.

14. Use of the isolated inclusion body according to any of the claims 1-6 and 10-12, as a tissue regenerator.

15. Isolated inclusion body according to any of the claims 1-6, 10-12, to be used as a medicinal drug.

16. Bacterial cell comprising the inclusion body as defined in any of the claims 1-6.

17. Bacterial cell according to claim 16, where the bacterial cell is *Escherichia coli*.

18. Bacterial cell according to claim 17, where the E. coli is selected from E. coli of a wild strain or a mutant strain.
